# EUROPEAN PATENT APPLICATION

(11) **EP 3 632 476 A1**
(43) Date of publication of application: **08.04.2020**
(21) Application number: 18198982.3
(22) Date of filing: 05.10.2018
(51) Int. Cl.: A61L 15/26, A61L 15/42

(54) **WOUND CONTACT SURFACE AND METHOD OF MANUFACTURE**

(71) Applicant: John J. Ryan (Sealing Products) Limited, Dunboyne A86 FP78, Co. Meath (IE)
(72) Inventor: RYAN, John, A86 FP78 (IE); GUCKIAN, Adrian, A86 FP78 (IE); MANTON, Jennifer Catherine, A86 FP78 (IE); BYRNE, Alan, A86 FP78 (IE); O'TOOLE, Danny, A86 FP78 (IE)
(74) Representative: Definition IP Limited

(57) **Abstract**

The present invention relates to a wound contact surface for a wound dressing. The wound contact surface can be incorporated into a wound dressing, such as a foam dressing, to provide a non-adherent layer between the dressing and the skin/wound. In aspects, the invention also relates to methods of preparation of the contact surface and dressings comprising same. The wound care surface and dressings comprising same have particular utility in the treatment of burns and blistering conditions such as epidermolysis bullosa.

## Description

### Technical Field

The present invention relates to a wound contact surface for a wound dressing. The wound contact surface can be incorporated into a wound dressing, such as a foam dressing, to provide a non-adherent layer between the dressing and the wound and, on removal to protect newly-formed skin and reduce trauma to the surrounding area. In aspects, the invention also relates to methods of preparation of the wound contact surface and wound dressings comprising the wound contact surface.

### Background

The skin is the largest organ in the human body, and performs protective, regulatory and sensory functions. Its primary role, however, is to act as a protective barrier against external forces, such as mechanical impact, pressure, temperature, pathogenic organisms, UV radiation and harmful chemicals. Injury can affect all of the functions of the skin and trigger a complex wound healing process.

Cutaneous wound healing is a major concern in medical fields, with more than 2.2 million wounds in adults recorded annually by the NHS, attributable to burns, ulcers, abscesses and trauma (Guest J.F., Ayoub N., Mcllwraith T. et al., "Health economic burden that wounds impose on the National Health Service in the UK", BMJ Open 2015;5). As a consequence, healthcare professionals have access to a large variety of wound dressings, with over 1,000 wound care products currently on the market worldwide. Within the NHS alone, healthcare workers have access to over 100 foam-based wound care products.

The primary contact surface of wound dressings, and particularly those used for chronic exuding wounds, must generally adhere sufficiently to the skin to form a fluid-tight seal around the wound, to ensure that no leakage of the exudate occurs leading to maceration of the peri-wound skin. At the same time, however, if the adhesion is too strong, trauma to the wound and the surrounding skin can occur upon removal. Trauma caused by the removal of wound dressings can delay or even reverse healing, and exacerbate wound pain. As well as causing additional stress and discomfort to patients, this can lengthen the term of care and increase the cost to healthcare providers.

Acrylic adhesives are often used in the primary contact surface of wound dressings, for instance in the form of an adhesive border, as they form a strong, secure seal with the skin. However, acrylic adhesives exhibit high levels of "tack" - defined as the instantaneous level of adhesion when a dressing is applied to the skin (Rippon et al. "Skin adhesives and their role in wound dressings"; Wounds UK (2007) Vol. 3(4): pp. 76-86.). This high level of tack makes acrylic-based wound dressings difficult to remove without causing trauma to the wound or to the surrounding area. Acrylic adhesives also leave an undesirable residue on the skin which can lead to skin irritation.

Polyurethane foam dressings, including both aromatic and aliphatic foams, exhibit excellent fluid absorption and retention characteristics, making them particularly desirable for wound care products. However, newly-formed skin can mesh into these foams during healing, a process which can be exacerbated by the high absorbency of the foam. This delicate, newly-formed skin may then be disturbed or even stripped upon removal of the dressing which causes trauma to the wound and lengthens the overall healing time of the wound.

In an effort to address this issue, absorbent wound care dressings often incorporate low-adherent wound contact surfaces, which are designed to prevent the absorbent layer of the dressing from sticking to the wound, thereby reducing the level of trauma on application and upon removal. Often these wound contact layers are formed of non-breathable materials, which are hole-punched, forming a lattice-type structure to allow absorption of exudate into the foam and air to penetrate to the wound bed. Dressings incorporating low-adherent wound contact surfaces are particularly suitable for use with superficial wounds such as those caused by burns, skin tears and blistering diseases and for immunocompromised and paediatric patients.

Among known wound contact surfaces are those formed of soft silicone. Soft silicone wound contact layers create a seal between the dressing and the wound, and ensure that exudate is absorbed into the dressing, thereby preventing leakage. However, silicones are expensive, are not breathable, tend to be difficult to functionalise and often cannot be gamma sterilised, limiting their application in certain clinical areas.

It is an aim of the invention to obviate or mitigate one or more of the disadvantages associated with the prior art. Ideally, it would be advantageous to provide a wound contact surface or layer which can provide a non-adherent layer between the dressing and the wound, restricting growth of new skin into the dressing and facilitating removal from the body with minimal trauma to the wound or surrounding skin. A wound contact surface or layer that is biocompatible, and/or breathable, and/or non-allergenic, and/or gamma sterilisable, and/or readily functionalised would be particularly advantageous. A simple and/or low-cost method of manufacturing the wound contact layer would also be beneficial.

### Summary of the Invention

According to the present invention there is provided a method of preparing a wound contact surface for a wound dressing, the method comprising coating a polyurethane formulation onto a wound dressing to form a surface, wherein the polyurethane formulation is a polyurethane having a peel adhesion of 0 N when measured in accordance with ISO 29862-2018 method 1.

According to another aspect of the present invention there is provided a wound contact surface for a wound dressing, wherein the wound contact surface is a surface or layer of a polyurethane formulation having a peel adhesion of 0 N when measured in accordance with ISO 29862-2018 method 1.

According to another aspect of the present invention there is provided a wound dressing comprising a wound contact surface and an absorbent layer, wherein the wound contact surface is a surface or layer of a polyurethane formulation having a peel adhesion of 0 N when measured in accordance with ISO 29862-2018 method 1.

Various further features and aspects of the invention are defined in the claims.

### Brief Description of the Drawings

Embodiments of the present invention will now be described by way of example only with reference to the accompanying drawings where, like parts are provided with corresponding reference numerals and in which:
Figure 1 shows a schematic of a wound contact surface coated on an absorbent foam according to an embodiment of the invention;
Figure 2 shows schematics of alternative wound contact surfaces according to embodiments of the invention;
Figure 3 illustrates the results of peel tests for aliphatic and aromatic polyurethane foam dressings comprising the wound contact surface versus those foam dressings without the wound contact surface; and
Figure 4 illustrates the results of absorption and retention testing for foam dressings comprising the wound contact surface versus foam dressings without the wound contact surface.

### Detailed Description

The present invention relates to a method of preparing a wound contact surface for a wound dressing, the method comprising coating a polyurethane formulation onto a wound dressing to form a surface, wherein the polyurethane formulation has a peel adhesion of 0N measured in accordance with ISO 29862-2018 method 1.

The surface may be a non-continuous surface.

By "non-continuous surface" it is meant that the polyurethane formulation is coated onto the wound dressing in a pattern which comprises one or more gaps or non-coated areas between the coated area(s) of the surface. These gaps or non-coated areas allow the exudate to be absorbed into the foam and facilitates the movement of air through to the wound bed while the dressing is *in situ*. For instance, the pattern may comprise a repeat series of coated dots, shapes or patterns, or one or more lines of coated formulation.

Alternatively, the surface may be a continuous surface.

The formulation is a polyurethane having a peel adhesion of 0N measured in accordance with ISO 29862-2018 method 1.

Polyurethanes are an important class of adhesives which, due to their versatility, are used in a wide variety of applications. Polyurethane adhesives are formed from isocyanates and polyols, which react to form the urethane (-NH-C(=O)-O-) backbone. The properties of polyurethane adhesives, and in particular their adhesive strength and tack, can be tailored to the application of interest as would be understood by one skilled in the art. Commercial polyurethane adhesives are commonly sold with directions for adjusting the adhesion level from low to high adhesion, depending on the intended use. According to the invention, the polyurethane formulation may be based on a polyurethane adhesive formulation, in which the adhesion has been controlled to give a peel adhesion value of 0 when measured according to ISO 29862-2018 method 1. Thus, the adhesive strength of the polyurethane formulation has been adjusted to a minimal or zero level, and outside the normal adhesive ranges. This adhesive level can be measured, for example, by ISO 29862-2018, method 1. When this method is performed, the polyurethane formulation will not adhere to the stainless-steel plate, thereby indicating a zero value for the peel adhesion of the formulation.

Advantageously, polyurethanes are biocompatible, non-allergenic, and gamma-sterilisable, making them particularly suitable for use in wound care applications. Unlike other known wound contact surface layer materials, polyurethane formulations have high moisture vapour transmission rates (MVTR), with reported MVTRs of up to 2,400 g/(day * m2) at a thickness of 110 µm. This breathability is very beneficial when used as a wound contact surface, as it allows air to circulate around the wound bed, which can further promote healing. Additionally, water vapour from the wound/exudate is able to permeate the dressing and evaporate into the atmosphere. This creates additional absorbency in the foam dressing, which then allows more exudate to be absorbed.

Additionally, polyurethanes are readily available, and medical grade polyurethane adhesives are already known for use in wound care products, albeit for adhesive applications. It has surprisingly been found that non-adhesive polyurethanes, in which the adhesive strength has been tailored, or dialled down to 0, can be used to provide a non-adherent layer or surface in wound care dressings. This non-adherent layer provides a simple and effective means of limiting ingrowth or formation of new skin into the absorbent dressing, reducing the peel force required to remove the absorbent wound dressing, thereby avoiding trauma to the wound and surrounding skin upon removal. This can significantly assist and speed up the wound healing process.

According to the invention, the polyurethane formulation has a peel adhesion of 0N measured in accordance with ISO 29862-2018 method 1, with the formulation coated on 180 gsm Bristol paper. The adhesion of the polyurethane can be controlled by manipulation of the components making up the polyurethane.

For instance, the polyurethane formulation may be a polyether-based polyurethane.

In an embodiment, the polyurethane formulation is made using a two-component polyurethane formulation, typically supplied as a two-component polyurethane adhesive. Two component polyurethane adhesives are known, and usually comprise of a first component containing diisocyante/isocyanate prepolymers, and a second component containing polyols (and optionally amine/hydroxyl chain extenders). The two components are reacted - often in the presence of a catalyst - to form the adhesive formulation. The level of adhesive strength of the polyurethane can be readily controlled, based on the intended use, by appropriate adjustment of the component ratios, as would be understood by a person skilled in the art. In the present invention, however, the level of adhesive strength of the polyurethane is adjusted to be non-adhesive, i.e. to give a no tack, non-adherent formulation.

In an embodiment, the two-component polyurethane adhesive comprises Baymedix™ AP501.

In an embodiment, the two-component polyurethane adhesive comprises Baymedix™ AR602.

In an embodiment, the two-component adhesive comprises Baymedix™ AR602 and Baymedix™ AP501. When the two-component polyurethane adhesive comprises Baymedix™ AR602 and Baymedix™ AP501, the components may be added in a ratio of from 1:0.15 to 1:0.18.

In an embodiment, when the two-component adhesive comprises Baymedix™ AR602 and Baymedix™ AP501, the components may be added in a ratio of from 1:0.15 to 1:0.25, or from 1:0.15 to 1:0.18.

According to the present invention, the polyurethane formulation is coated
onto a wound dressing or wound dressing layer to form a surface. The formulation can be coated onto the dressing by any suitable method. For instance, coating can be performed by printing, deposition or patterning.

In an embodiment of the invention, coating is performed using a printing technique. When the polyurethane formulation is coated on the wound dressing or wound dressing layer by printing, the formulation can be printed by methods such as screen printing, rotary screen printing, flexo printing, laminating, gravure or inkjet printing.

In an embodiment, the formulation is printed by rotary screen printing or gravure printing. Alternatively, coating can be performed using deposition or patterning techniques. Suitable deposition or patterning techniques for use in coating the polyurethane formulation onto the wound dressing or wound dressing layer, include, but are not limited to, Langmuir Blodgett, spin coating, dip coating, atomic layer deposition, solution deposition and sputtering techniques.

Once the polyurethane formulation is coated onto the dressing it is allowed to cure. In an embodiment, the polyurethane formulation is allowed to cure at a temperature between 75 °C and 130 °C. In an embodiment, the polyurethane formulation is allowed to cure for a period of time of from 3 minutes to 20 minutes. Alternatively, the formulation is allowed to cure under IR light from 30 seconds to 15 minutes.

In an embodiment, the surface is a non-continuous surface. In this embodiment, the pattern of the coating or printing is also not particularly limited, once it comprises sufficient gaps or non-coated regions to allow movement of air and/or absorption of the exudate into the foam through this surface. Typically the non-coated area could vary anywhere from 1 % to 99%, or from 5 to 95% of the total surface area. This would be readily ascertained by one skilled in the art, and can take into account factors such as the intended application of the dressing (and the amount of exudate expected), and the performance requirements of the dressing. For instance, up to 10% of the surface may be non-coated. In another embodiment, up to 20%, up to 30%, up to 40% or up to 50% of the surface may be non-coated.

Preferably the pattern is a repeating pattern.

Alternatively, the surface is a continuous surface. This embodiment may be suitable for non-exuding wounds, such as burns. Advantageously, the high breathability of the polyurethane formulation allows movement of air around the wound bed, and the lack of exudate means that gaps in the coated surface are not necessarily required.

According to the invention, the polyurethane formulation is coated onto a wound dressing layer. This layer can be a surface of the wound dressing itself, or can be a separate layer or substrate which is subsequently affixed or laminated to the wound dressing to provide a wound contact surface.

In an exemplary embodiment of the invention, the polyurethane formulation has been formed using a two-component polyurethane adhesive formulation, the adhesive properties of which have been controlled, to provide a low tack or no tack formulation, indicated by a peel adhesion value of 0 N.

As would be understood by a person skilled in the art, the polyurethane formulation can be modified, for instance using rheological modifiers etc, to ensure compatibility with the chosen coating or printing technique, so long as the additives do not affect the peel adhesion characteristics of the polyurethane formulation. Alternatively, the polyurethane formulation may consist of the polyurethane only.

In an embodiment, therefore, there is provided a method of preparing a wound contact surface for a wound dressing, the method comprising:
preparing a polyurethane formulation having a peel adhesion of 0 N when measured in accordance with ISO 29862-2018 method 1;
coating the polyurethane formulation onto a wound dressing or wound dressing layer to form a surface thereon; and
allowing the surface to cure.

Curing may be performed at a temperature of from 75°C to 130°C for from 3 minutes to 30 minutes or under IR light for 30 seconds to 15 minutes.

The surface may be a continuous or non-continuous surface.

In an embodiment, the polyurethane formulation may be printed onto the wound dressing or wound dressing layer.

According to an aspect of the present invention there is provided a wound contact surface for a wound dressing, wherein the wound contact surface is a continuous or non-continuous surface of a polyurethane formulation having a peel adhesion of 0 N when measured in accordance with ISO 29862-2018 method 1.

The continuous or non-continuous surface of polyurethane formulation is a cured layer of the polyurethane formulation.

According to an aspect of the present invention there is provided a wound dressing comprising a wound contact surface, wherein the wound contact surface is a continuous or non-continuous surface of a polyurethane formulation having a peel adhesion of 0 N when measured in accordance with ISO 29862-2018 method 1.

The wound dressing may be a laminate or multi-laminate structure. In an embodiment, the wound dressing comprises an absorbent layer. The wound dressing may comprise or consist of, for instance, an absorbent foam. Suitable absorbent foams include both aromatic and aliphatic foams. The aromatic and/or aliphatic foams may have antimicrobial agents incorporated therein.

In an embodiment, the wound dressing comprises a polyurethane foam. The polyurethane foam may be an aliphatic polyurethane foam or an aromatic polyurethane foam. In an embodiment, the polyurethane foam is an aliphatic polyurethane foam. In an alternative embodiment, the polyurethane foam is an aromatic polyurethane foam.

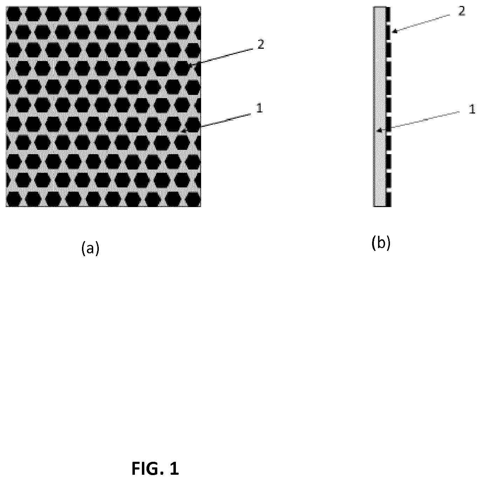

A wound dressing according to the present invention is illustrated in Figure 1(a). The wound dressing comprises a layer of polyurethane foam (1), onto which a wound contact surface has been coated directly. The wound contact surface comprises a series of hexagonal dots (1), interspersed or separated by non-coated regions (2), through which the exudate can enter the absorbent foam, and which allows air to circulate around the wound bed. In the embodiment shown, the non-coated regions account for approximately 40% of the surface of the dressing. The wound contact surface may be surrounded by a border of adhesive, such as an acrylic adhesive (not shown). Figure 1(b) is a side-view of the dressing, showing coating of the hexagonal dots of polyurethane adhesive (2) on the polyurethane foam surface (1).

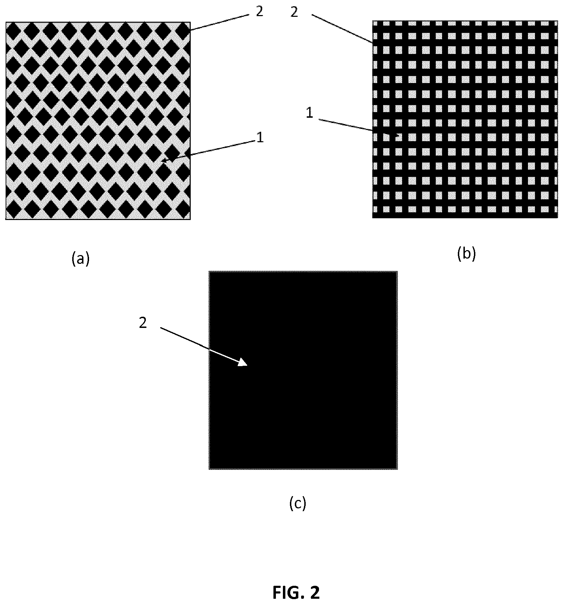

Alternative embodiments of the dressing are shown in Figure 2. In Figure 2(a), the polyurethane formulation has been coated as a series of diamond shapes, while in Figure 2(b), the polyurethane formulation has been coated as a series of interweaving lines with non-continuous gaps in the printed surface. A skilled person would recognise that the pattern of the coating is not limited, once the contact surface comprises sufficient gaps or non-coated regions in the pattern to allow absorption of any expected exudate into the dressing and movement of air around the wound bed while the dressing is in place on the wound. Preferably, the polyurethane formulation is coated in a repeating pattern, to ensure a consistent peel strength for removal of the dressing in order to further minimise trauma to the wound or the surrounding skin. Again, in these embodiments, the wound contact surface may be surrounded by a border of adhesive, such as an acrylic adhesive (not shown). Figure 2(c) shows an alternative embodiment in which the polyurethane formulation has been coated as a continuous layer or surface. This embodiment may be suitable for non-exuding wounds.

The invention will now be described by way of reference to the following examples, which are intended to be illustrative only.

### Examples

### Example 1: Preparation of polyurethane formulation

A commercially-available two component polyurethane adhesive was purchased from Baymedix™ and prepared at a ratio of polyol:isocyanate of 1:0.165 as follows: 79.90 g of the polyol component (Baymedix™ AR602) was added to a suitable vessel.

Separately, a catalyst was prepared by mixing 0.31 g of Coscat 83 and 0.19 g of BorchiKat 0761 to prepare a 1.0:0.6 mix, which was then added to the polyol component (a loading of 1% catalyst).

The components were then mixed using a Hauschild DAC 400.1 FVZ Speedmixer at 2,750 rpm for 30 seconds.

16.55 g of Baymedix AP501 was added to the vessel, and the components mixed again at 2,750 rpm for 30 seconds.

The polyurethane formulation was used immediately.

### Example 2: Measurement of peel adhesion of polyurethane formulation

The peel adhesion of the polyurethane formulation was measured in accordance with ISO: 29862-2018. Method 1: "Self-adhesive tapes. Measurement of peel adhesion from stainless steel at an angle of 180°". In brief, the polyurethane formulation was coated onto 180 gsm Bristol Paper. A 25 x 2.5 cm sample of the coated paper was cut and adhered to a 20 x 5 cm stainless steel plate. The peel adhesion was measured at 180 ° using a Zwick Z0.5 peel adhesion testing instrument with a grip to grip separation of 185 mm and a speed of 585 mm/min.

The coated Bristol paper would not adhere to the plate, and so a 0 N peel adhesion value was recorded. Samples were measured in quadruplicate, and a 0 N value was recorded for all samples.

### Example 3: Printing of contact layer

The polyurethane formulation prepared in Example 1 above was screen-printed onto aliphatic and aromatic polyurethane foam dressings as follows:
The polyurethane formulation was wiped, in a single sweep, using a squeegee over a set-patterned screen print (in this case a pattern comprising a series of evenly-spaced hexagons as in Figure 1). The foam was then removed, and cured at 100 °C in a Binder Benchtop Oven FED 115 fan-assisted oven for 7 minutes.

### Example 4: Peel testing

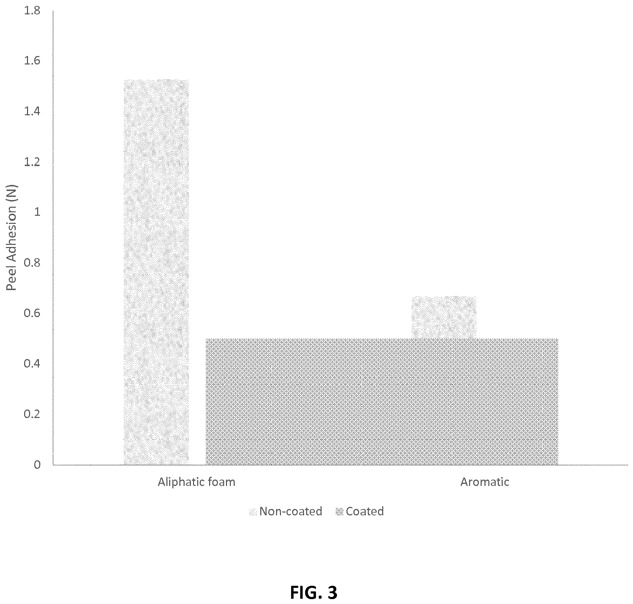

In order to assess the effect of the wound contact surface on the peel force required to remove the dressing, aliphatic and aromatic polyurethane foam dressings comprising a contact surface as printed in Example 2, and the same foam dressings without this contact surface were tested as follows:
A simulated wound exudate solution was prepared by adding 5 drops of a solution of red dye (Neutral Red dye dissolved in deionised water from Sigma Aldrich CAS Number 553-24-2) to 250 ml of a 40% (w/v) mix of gelatine (Sigma Aldrich Cat. # 48723) in hot (70°C) deionised water. 30 ml of the solution was then poured into a plastic box (20 x 5 cm) and left to settle for 3 minutes. A 25 x 2.5 cm sample of the foam dressing to be tested was placed gently on top of the drying gelatine mix. The sample was then left at room temperature (22°C) for 2 hours. The peel force was then measured at 180 ° using a Zwick Z0.5 peel adhesion testing instrument with a grip to grip separation of 185 mm and a speed of 585 mm/min. All samples were measured in quadruplicate (aliphatic foam dressing) and triplicate (aromatic foam dressing). The results of the peel tests for the dressings comprising the wound contact layer and those without the wound contact layer are shown in Table 1 and illustrated in Figure 3:

**Table 1: Peel testing of dressings comprising wound contact layer versus dressings with no wound contact layer**

| Dressing | Aliphatic foam dressing | | Aromatic foam dressing | |
|---|---|---|---|---|
| Sample # | Without wound contact surface Peel adhesion(N) | With wound contact surface Peel adhesion (N) | Without wound contact surface Peel adhesion (N) | With wound contact surface Peel adhesion (N) |
| 1 | 1.69 | 0.683 | 0.746 | 0.273 |
| 2 | 1.62 | 0.444 | 0.525 | 0.257 |
| 3 | 1.48 | 0.444 | 0.670 | 0.166 |
| 4 | 1.34 | 0.410 | - | - |
| Average | 1.53 | 0.50 | 0.670 | 0.232 |

The results in table 1 show that the aliphatic foam dressings comprising the wound contact layer required a peel force of 0.50 N on average, versus a peel force of 1.53 on average for the dressings which did not comprise the contact layer. For aromatic foam dressings comprising the wound contact layer, a peel force of 0.232 N was required on average, versus a peel force of 0.670 for those aromatic foam dressings which did not comprise the contact layer.

### Example 5: Absorbance and retention testing

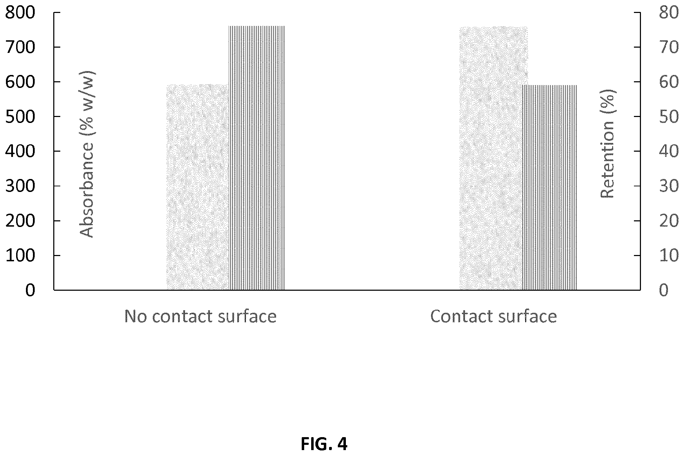

In order to assess whether the incorporation of the wound contact surface affected the absorbance of the foam dressing, absorbance and retention tests were carried out according to BS:EN 13726 Aspects of Absorbency (part 1; 3.2). In brief, a 5 x 5 cm aliphatic foam sample was weighed and placed into Test Solution A (Section 3.2.2.3 of BS:EN 13726 - 8.298g sodium chloride and 0.368g calcium chloride dihydrate dissolved in 1L deionised water) to simulate a wound environment for 30 minutes at 37°C. The sample was then suspended vertically by the corner of the dressing for 30 seconds, before being re-weighed to assess absorbance. Fluid retention was then measured using the saturated 5 x 5 cm foam sample from the experiment above. The sample was subjected to a 6 kg weight for 30 seconds, while sitting on a plinth just wide enough to allow the foam sample to lay flat on it. The weight was then removed, and the sample re-weighed. The results of the absorbance and retention testing are shown in Table 2, and illustrated in Figure 4.

**Table 2: Absorbance and retention testing of dressings comprising wound contact surface versus dressings with no wound contact layer.**

| | With wound contact surface | | Without wound contact surface | |
|---|---|---|---|---|
| Sample # | Absorbance (% w/w) | Retention (%) | Absorbance (% w/w) | Retention (%) |
| 1 | 709 | 60 | 628 | 72 |
| 2 | 804 | 53 | 572 | 77 |
| 3 | 769 | 65 | 585 | 79 |
| Average | 761 | 59 | 595 | 76 |

These results demonstrate that there is no significant effect on the absorbance and retention characteristics of the foam dressing when the wound contact surface is added.

Accordingly, the wound contact surface of the present invention, when applied to a foam dressing, demonstrably reduces the peel force required to remove the dressing from a surface, without affecting the absorbance or retention characteristics of the dressing. No visible residue was observed post-removal of the dressing in the tests carried out above. The wound contact surface therefore demonstrates suitable characteristics for use in wound care dressings, particularly where low trauma removal is required, such as for wounds arising from burns, and blistering diseases such as epidermolysis bullosa etc. The wound contact surface can be applied to a dressing by simple coating methods, such as printing, using readily available materials, allowing for facile, cost-effective manufacturing. Advantageously, the wound contact surface is biocompatible, breathable, non-allergenic and can be gamma-sterilised.

All of the features disclosed in this specification (including any accompanying claims, abstract and drawings), and/or all of the steps of any method or process so disclosed, may be combined in any combination, except combinations where at least some of such features and/or steps are mutually exclusive. Each feature disclosed in this specification (including any accompanying claims, abstract and drawings) may be replaced by alternative features serving the same, equivalent or similar purpose, unless expressly stated otherwise. Thus, unless expressly stated otherwise, each feature disclosed is one example only of a generic series of equivalent or similar features. The invention is not restricted to the details of the foregoing embodiment(s). The invention extends to any novel one, or any novel combination, of the features disclosed in this specification (including any accompanying claims, abstract and drawings), or to any novel one, or any novel combination, of the steps of any method or process so disclosed.

With respect to the use of substantially any plural and/or singular terms herein, those having skill in the art can translate from the plural to the singular and/or from the singular to the plural as is appropriate to the context and/or application. The various singular/plural permutations may be expressly set forth herein for sake of clarity.

It will be understood by those within the art that, in general, terms used herein, and especially in the appended claims are generally intended as "open" terms (e.g., the term "including" should be interpreted as "including but not limited to," the term "having" should be interpreted as "having at least," the term "includes" should be interpreted as "includes but is not limited to," etc.). It will be further understood by those within the art that if a specific number of an introduced claim recitation is intended, such an intent will be explicitly recited in the claim, and in the absence of such recitation no such intent is present. For example, as an aid to understanding, the following appended claims may contain usage of the introductory phrases "at least one" and "one or more" to introduce claim recitations. However, the use of such phrases should not be construed to imply that the introduction of a claim recitation by the indefinite articles "a" or "an" limits any particular claim containing such introduced claim recitation to embodiments containing only one such recitation, even when the same claim includes the introductory phrases "one or more" or "at least one" and indefinite articles such as "a" or "an" (e.g., "a" and/or "an" should be interpreted to mean "at least one" or "one or more"); the same holds true for the use of definite articles used to introduce claim recitations. In addition, even if a specific number of an introduced claim recitation is explicitly recited, those skilled in the art will recognize that such recitation should be interpreted to mean at least the recited number (e.g., the bare recitation of "two recitations," without other modifiers, means at least two recitations, or two or more recitations).

It will be appreciated that various embodiments of the present disclosure have been described herein for purposes of illustration, and that various modifications may be made without departing from the scope of the present disclosure. Accordingly, the various embodiments disclosed herein are not intended to be limiting, with the true scope being indicated by the following claims.

## Claims

1. A method of preparing a wound contact surface for a wound dressing, the method comprising:
coating a polyurethane formulation onto a wound dressing or wound dressing layer to form a surface, wherein the polyurethane formulation has a peel adhesion of 0N measured in accordance with ISO 29862-2018 method 1.

2. A method of preparing a wound contact surface as claimed in claim 1, wherein the coating is performed by printing, deposition or patterning onto the wound dressing or wound dressing layer.

3. A method of preparing a wound contact surface as claimed in claim 1 or claim 2,
wherein the surface is a continuous or a non-continuous surface.

4. A method of preparing a wound contact surface as claimed in claim 2, wherein the polyurethane formulation is printed onto the wound dressing or wound dressing layer by screen printing, rotary screen printing, flexo printing, laminating, gravure or inkjet printing.

5. A method of preparing a wound contact surface as claimed in any preceding claim,
wherein the wound dressing is a foam dressing.

6. A method of preparing a wound contact surface as claimed in claim 5, wherein the foam dressing is a polyurethane foam.

7. A method of preparing a wound contact surface as claimed in any preceding claim, comprising:
preparing a polyurethane formulation having a peel adhesion of 0 N when measured in accordance with ISO 29862-2018 method 1;
coating the polyurethane formulation onto a wound dressing or wound dressing layer to form a surface thereon;
allowing the surface to cure.

8. A method of preparing a wound contact surface as claimed in claim 7, wherein curing takes place at a temperature of from 75°C to 130 °C for from 3 minutes to 30 minutes or under IR light for 30 seconds to 15 minutes.

9. A method of preparing a wound contact surface as claimed in any preceding claim,
wherein the polyurethane formulation having a peel adhesion of 0 N is preparing by controlling the ratios of the constituent groups of the polyurethane.

10. A method of preparing a wound contact surface as claimed in any preceding claim,
wherein the polyurethane formulation is a polyether-based polyurethane.

11. A wound contact surface for a wound dressing, wherein the wound contact surface is a continuous or non-continuous surface of a polyurethane formulation having a peel adhesion of 0 N when measured in accordance with ISO 29862-2018 method 1.

12. A wound contact surface as claimed in claim 11, wherein the polyurethane formulation is a polyether-based polyurethane.

13. A wound dressing comprising a wound contact surface as claimed in claim 11 or claim 12.

14. A wound dressing as claimed in claim 13 comprising a polyurethane foam.

15. A wound dressing as claimed in claim 14, wherein the polyurethane foam is an aliphatic polyurethane foam or an aromatic polyurethane foam, optionally wherein the polyurethane foam is an aliphatic polyurethane foam.
